# EUROPÄISCHE PATENTSCHRIFT

(11) **EP 0 850 026 B1**
(45) Veröffentlichungstag und Bekanntmachung des Hinweises auf die Patenterteilung: **21.02.2001**
(21) Anmeldenummer: 96932439.1
(22) Anmeldetag: 14.08.1996
(51) Int. Cl.: A61B 19/00, A61B 6/04, A61B 6/08

(54) **GANZKÖRPER-STEREOTAXIEVORRICHTUNG**
WHOLE BODY STEREOTAXIC DEVICE
DISPOSITIF STEREOTAXIQUE POUR LE CORPS ENTIER

(30) Priorität: 14.08.1995 DE 19529867
(43) Veröffentlichungstag der Anmeldung: 01.07.1998
(73) Patentinhaber: Deutsches Krebsforschungszentrum Stiftung des öffentlichen Rechts, 69120 Heidelberg (DE)
(72) Erfinder: PASTYR, Otto, D-69181 Leimen (DE); SCHLEGEL, Wolfgang, D-69121 Heidelberg (DE)
(74) Vertreter: Castell, Klaus, Dr.-Ing.
(86) Internationale Anmeldenummer: DE9601521
(87) Internationale Veröffentlichungsnummer: WO9706743

(56) Entgegenhaltungen:
- WO-A-90/00372
- US-A- 3 859 982
- US-A- 4 262 204
- US-A- 5 242 455
- US-A- 5 281 232

## Beschreibung

Die vorliegende Erfindung betrifft eine Ganzkörper-Stereotaxievorrichtunggemäß dem Oberbegriff des Anspruchs 1. Eine derartige Vorrichtung ist bereits grundsätzlich aus der US-5,281,232 bekannt.

Die vorbekannte Ganzkörper-Stereotaxievorrichtung besitzt eine Grundplatte und zwei sich daran anschließende randseitig befestigte und gekrümmte Randleisten. Die beiden Randleisten sind durch sich über die Grundplatte erstreckende Bügel verbunden. Diese Ganzkörper-Stereotaxievorrichtung ist nachteilig, da die Möglichkeit fehlt, Punkte im Bereich der Mitte der Grundplatte exakt zu lokalisieren. Dadurch kann auch der auf der Grundplatte aufliegende Körper eines Patienten in seinem an die Grundplatte angrenzenden Bereich nur ungenau lokalisiert werden. Die stabförmigen Kalibrierungsstäbe der genannten Druckschrift sind ferner unhandlich und erlauben lediglich eine Kalibrierung des Bügels, an dem sie befestigt sind. Ferner fehlen in der Anordnung dieser Druckschrift Einrichtungen zur Zielpositionierung der gesamten Vorrichtung für eine spätere Bestrahlung des Patienten. Schließlich sind zwei benachbarte Bügel in ihrer relativen Positionierung zueinander nicht exakt definiert.

Aufgabe der vorliegenden Erfindung ist die Angabe einer Ganzkörper-Stereotaxievorrichtung, welche die Nachteile des genannten Standes der Technik vermeidet und insbesondere eine exakte Lokalisierung auch im Bereich der Grundplatte und daran angrenzenden Bereiche gestattet. Dabei sollen bequem handhabbare Kalibrierungseinrichtungen angegeben werden und die relative Positionierung benachbarter Bügel soll erleichtert werden.

Gemäß der vorliegenden Erfindung ist eine Ganzkörper-Stereotaxievorrichtung nach dem Oberbegriff des Anspruchs 1 vorgesehen, die dadurch gekennzeichnet ist, daß die Bügel durch mindestens einen Haltestab verbunden sind, der zur Halterung chirurgischer Instrumente dient. Der Haltestab definiert einen eindeutigen Abstand zwischen den beiden benachbarten Bügeln, so daß die Lokalisierung von an dem Haltestab angebrachten chirurgischen Instrumenten erleichtert ist. Auch während der Betätigung der an dem Haltestab angebrachten chirurgischen Instrumente verändert sich der Abstand zwischen den beiden Bügeln nicht.

Gemäß einer Weiterbildung der Erfindung ist eine Ganzkörper-Stereotaxievorrichtung angegeben, die dadurch gekennzeichnet ist, daß an der Grundplatte eine Mittelleiste befestigt ist, die Bohrungen zur Aufnahme von Zubehör aufweist. An der Mittelleiste können insbesondere Kalibrierungseinrichtungen aufgenommen werden, die somit auch eine Kalibrierung im Bereich der Grundplatte gestatten.

Gemäß einem weiteren Aspekt der vorliegenden Erfindung ist eine Ganzkörper-Stereotaxievorrichtung nach Anspruch 3 vorgesehen, die dadurch gekennzeichnet ist, daß der Bügel eine Zieleinstellungseinrichtung für die spätere Bestrahlung des Patienten trägt. Die Zieleinstellungseinrichtung ermöglicht eine exakte Positionierung der gesamten Ganzkörper-Stereotaxievorrichtung bezüglich eines Bestrahlungssystems.

Es ist bevorzugt, daß die Grundplatte Kohlenstoff-Fasern enthält. Diese tragen erheblich zur Stabilität der Grundplatte bei.

Bevorzugt ist, daß die Grundplatte Acrylschaum enthält. Dieser erhöht die Flexibilität der Kohlenstoff-Fasern.

Die Grundplatte weist in einer besonders bevorzugten Ausführungsform abwechselnd eine Lage Acrylschaum und eine Lage Kunststoff-Fasern in Sandwichform auf. Damit werden besonders zufriedenstellende Ergebnisse bezüglich Leichtigkeit, Handhabbarkeit und Flexibilität der Grundplatte erzielt.

Bevorzugt ist an einem Bügel oder an der Mittelleiste eine Kalibriereinrichtung befestigt, die einen definierten Bezugspunkt in der Computertomographie liefert. Die Kalibriereinrichtung weist bevorzugt eine im Querschnitt dreieckige Lokalisationsplatte auf, in der Metalldrähte aufgenommen sind. Die Metalldrähte liefern einen Referenzpunkt oder eine Referenzlinie in der Computertomographie, anhand derer die Stereotaxievorrichtung kalibrierbar ist.

Auf der Grundplatte ist bevorzugt eine Fixiereinrichtung für den Patienten, insbesondere eine Vakuummatratze aufgenommen. Diese dient zur eindeutigen und lagestabilen Fixierung des Patienten bezüglich der Stereotaxievorrichtung. Die Vakuummatratze wird um den Patienten geschlagen und sodann evakuiert, um sie zu versteifen.

Es sind bevorzugt Haltebleche vorgesehen, die zwischen Randleiste und Fixiereinrichtung eingesetzt werden, um die Fixierung des Patienten zu erleichtern.

Für eine exakte Positionierung ist es bevorzugt, daß eine Lokalisationsplatte an dem Bügel und eine Lokalisationsplatte an der Mittelleiste befestigt sind, wobei die beiden Lokalisationsplatten in senkrechter Richtung miteinander fluchten. Dadurch läßt sich die Positionierung der Ganzkörper-Stereotaxievorrichtung exakt vornehmen.

An dem Haltestab ist bevorzugt ein chirurgisches Instrument zur Fixierung der Wirbelsäule angebracht. Der Haltestab ist in seiner Position bezüglich der beiden Bügel exakt definiert und ermöglicht eine eindeutige und positionsstabile Fixierung eines chirurgischen Instruments zur Fixierung der Wirbelsäule des Patienten, so daß der Patient eindeutig und unverrückbar zur Stereotaxievorrichtung positioniert ist.

Der Bügel ist besonders bevorzugt zweigeteilt, um ihn ggf. halbseitig zu demontieren. Dadurch ist das Spektrum seiner Einsatzmöglichkeiten erhöht.

Bevorzugt sind drei Zieleinstellungsvorrichtungen an dem Bügel befestigt. Dies gestattet eine wirksame Positionierung der beiden Randbereiche und des mittleren Bereichs der Ganzkörper-Stereotaxievorrichtung.

An dem Bügel ist besonders bevorzugt ein Hilfsbügel befestigt, der weitere medizinische Therapie- oder Diagnosemöglichkeiten, insbesondere für die Biopsie eröffnet. Bevorzugt ist an dem Hilfsbügel ein verschieblich gelagerter Läufer angebracht. Ein derartiger Läufer ermöglicht eine rasche Zieleinstellung entlang der Bahn des Hilfsbügels. Weitere Vorteile, Merkmale und Anwendungsmöglichkeiten der Erfindung ergeben sich aus der nachfolgenden Beschreibung verschiedener Ausführungsbeispiele in Verbindung mit der Zeichnung.
- Fig. 1: ist eine perspektivische Ansicht einer Ganzkörper-Stereotaxievorrichtung gemäß der Erfindung.
- Fig. 2: ist eine Seitenansicht der Ausführungsform von Fig. 1 in Perspektive.
- Fig. 3: ist eine Ansicht eines zweigeteiltens Bügels der Erfindung.
- Fig. 4: ist eine Aufsicht auf die in der vorliegenden Erfindung eingesetzten Haltebleche.
- Fig. 5: ist eine perspektivische Ansicht eines mit einem Hilfsbügel versehenen Bügels.

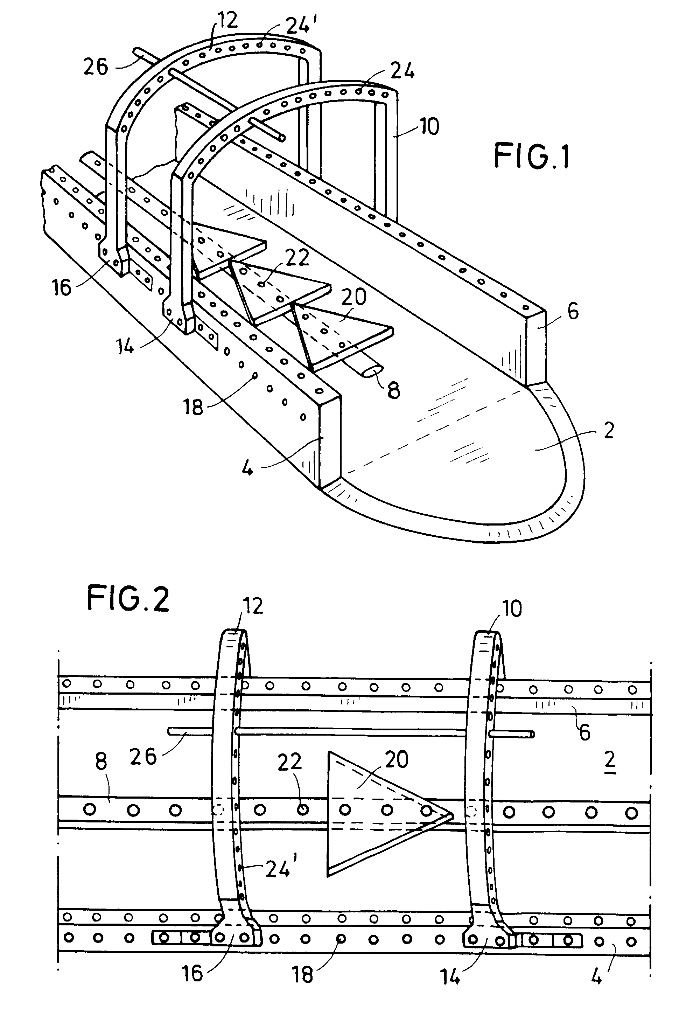

Die Ganzkörper-Stereotaxievorrichtung gemäß Fig. 1 und 2 weist eine Grundplatte 2 auf, die aus sandwichartig übereinandergelagerten Kohlenstoff-Fasern und Acrylschaumschichten gebildet ist. Randseitig sind auf der Grundplatte 2 Randleisten 4 und 6 angebracht, welche die Grundplatte seitlich abschließen und zur Halterung von Aufbauten bzw. von Zubehör dienen. Die Randleisten sind aus demselben Material gemacht wie die Grundplatte. Sie weisen seitliche Bohrungen 18 auf, die zum Anschluß von Aufbauten dienen. Etwa mittig ist in Längsrichtung auf der Grundplatte 2 eine Mittelleiste 8 befestigt, welche Sackbohrungen 22 zur Aufnahme von Zubehör aufweisen. Eine Vielzahl von Sackbohrungen 22 sind längs der Mittelleiste 8 verteilt angebracht.

An den beiden Randleisten 4 und 6 ist ein sich über die Grundplatte 2 erstrekkender Bügel 10 angeordnet, der in seiner Befestigungsposition zwei senkrechte Abschnitte und einen gekrümmten, die beiden senkrechten Abschnitte verbindenden Abschnitt aufweist, in dem Befestigungsbohrungen 24 gebildet sind. Der Bügel 10 ist mit einem Befestigungsteil 14 an der Randleiste 4 befestigt. Ein entsprechendes Befestigungsteil weist der Bügel 10 zur Befestigung an der Randleiste 6 auf.

Ein dem Bügel 10 entsprechender Bügel 12 ist hinter dem Bügel 10 in einem bestimmten Abstand an den Randleisten 4 und 6 derart befestigt, daß er sich im wesentlichen parallel zu dem Bügel 10 erstreckt. Der Bügel 12 ist mit einem Befestigungsteil 16 an der Randleiste 4 und einem entsprechenden Befestigungsteil (nicht gezeigt) an der Randleiste 6 befestigt. Den Befestigungsbohrungen 24 entsprechende Befestigungsbohrungen 24' sind in dem gekrümmten Abschnitt des Bügels 12 gebildet. In gegenüberliegenden Befestigungsbohrungen 24 und 24' der beiden benachbarten Bügel 10 und 12 ist ein Haltestab 26 aufgenommen, der zur Halterung von Zubehör, insbesondere für chirurgische Instrumente dient. In den Befestigungsbohrungen sind jedoch auch Kalibriereinrichtungen für die Kalibrierung der Stereotaxievorrichtung sowie Zieleinstellungseinrichtungen für die spätere Bestrahlung des Patienten anbringbar.

Auf der Mittelleiste 8 sind in der in Fig. 1 gezeigten Ausführungsform drei hintereinander angeordnete Kalibriereinrichtungen in Form von Lokalisationsplatten 20 befestigt. Die Lokalistationsplatten 20 haben im Querschnitt eine dreieckige Form und sind mit einem an der Dreieckspitze unterseitig gebildeten Zapfen in einer der Bohrungen 22 der Mittelleiste 8 aufgenommen. Fig. 2 zeigt die Ausführungsform der Fig. 1 in seitlicher perspektivischer Ansicht von links.

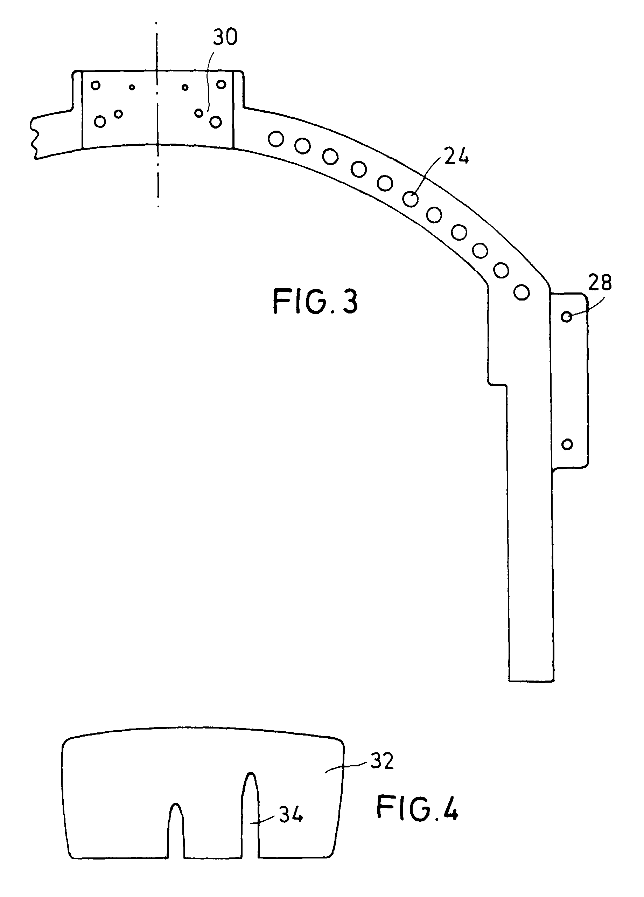

Fig. 3 zeigt den Aufbau und die Form eines Bügels 10 bzw. 12 der Erfindung. Der Bügel besitzt einen gerade und senkrecht verlaufenden Abschnitt, in dem in einem Vorsprung zwei gegenüberliegende Bohrungen 28 zum Anbringen einer Lokalisationsplatte 20 oder einer Zieleinstellungseinrichtung gebildet sind. An diesen Abschnitt schließt sich ein gekrümmter Abschnitt an, in dem die bereits vorstehend genannten Befestigungsbohrungen 24 zum Einbringen von Haltestäben 26 gebildet sind. Schließlich schließt sich an den gekrümmten Abschnitt ein Endteil an, in dem Befestigungsbohrungen 30 wiederum für eine Lokalisationsplatte 20 oder eine Zieleinstellungseinrichtung gebildet sind. Die senkrechte gestrichelte Mittellinie zeigt eine mögliche Teilungslinie des zweiteilig ausgebildeten Bügels. Der Bügel läßt sich in der zweigeteilten Form entweder einteilig verwenden oder zunächst zweiteilig montieren, wobei ein Teil sodann demontierbar ist.

Fig. 4 zeigt die Form eines Halteblechs 32, das zwischen Randleiste 4, 6 und einer Fixiereinrichtung für den Patienten, insbesondere einer Vakuummatratze angeordnet werden kann. Das Halteblech 32 ist bevorzugt gekrümmt und weist zwei Schlitze 34 zur Erhöhung der Flexibilität auf.

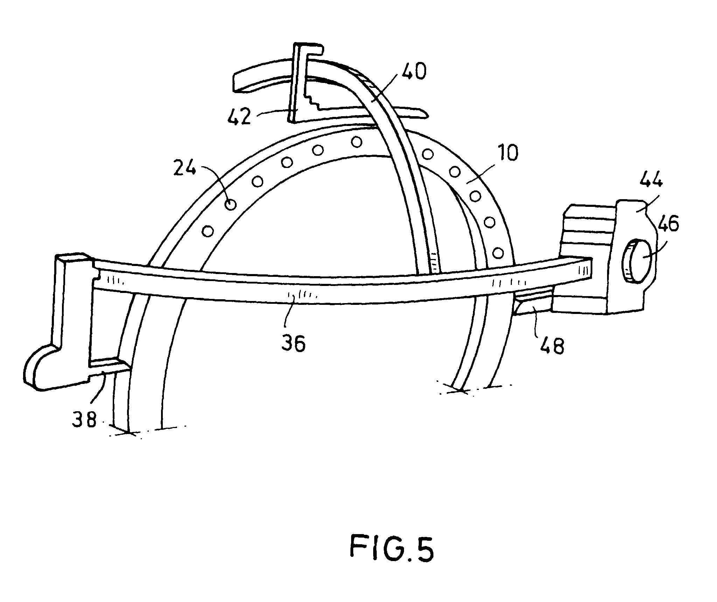

In Fig. 5 ist ein Bügel 10 gezeigt, der mit einem zusätzlichen quergelagerten Hilfsbügel 36, beispielsweise für Anwendungen in der Biopsie versehen ist. Der Hilfsbügel 36 ist mit zwei punktuellen Befestigungseinrichtungen 38, 48 an dem Bügel 10 befestigt und weist einen längs seiner Länge verschieblich gelagerten Läufer 44 auf, der an einer Rändelmutter 46 lösbar bzw. fixierbar ist. An dem Läufer 44 können verschiedene chirurgische Instrumente, insbesondere für die Biopsie befestigt werden. Der Querbügel 36 ist mit einem Aufbügel 40 versehen, der in einer an dem Bügel 10 befestigten oberen Halteeinrichtung 42 gehaltert ist. Wenn die Halteeinrichtung 42 gelöst wird, läßt sich der Querbügel 36 durch Verschiebung des Aufbügels 40 in seiner Höhe bezüglich des Bügels 10 einstellen und wieder fixieren. Der Querbügel 36 gestattet somit zusätzlich zu dem Haltestab die Anbringung einer Vielzahl weiterer chirurgischer Instrumente.

## Patentansprüche

1. Ganzkörper-Stereotaxievorrichtung mit
einer Grundplatte (2) zur Aufnahme eines Patienten,
zwei auf der Grundplatte (2) randseitig befestigten Randleisten (4, 6), welche die Grundplatte (2) seitlich abschließen und an denen die Grundplatte (2) überspannende Bügel (10, 12) befestigbar sind, wobei zwei einander gegenüberliegende Bügel (10, 12) an den Randleisten (4, 6) angebracht sind
dadurch gekennzeichnet, daß die Bügel (10, 12) durch mindestens einen Haltestab (26) verbunden sind, der zur Halterung chirurgischer Instrumente bestimmt ist.

2. Ganzkörper-Stereotaxievorrichtung nach Anspruch 1
dadurch gekennzeichnet, daß an der Grundplatte (2) eine Mittelleiste (8) befestigt ist, die Bohrungen (22) zur Aufnahme von Zubehör aufweist.

3. Ganzkörper-Stereotaxievorrichtung nach einem der Ansprüche 1 oder 2 dadurch gekennzeichnet, daß ein Bügel (10, 12) eine Zieleinstellungseinrichtung für eine spätere Bestrahlung des Patienten trägt.

4. Ganzkörper-Stereotaxievorrichtung nach einem der Ansprüche 1 bis 3, dadurch gekennzeichnet, daß die Grundplatte Kohlenstoff-Fasern enthält.

5. Ganzkörper-Stereotaxievorrichtung nach einem der vorhergehenden Ansprüche, dadurch gekennzeichnet, daß die Grundplatte Acrylschaum enthält.

6. Ganzkörper-Stereotaxievorrichtung nach Anspruch 4 und 5, dadurch gekennzeichnet, daß die Grundplatte abwechseind eine Lage Acrylschaum und eine Lage Kohlenstoff-Fasern in Sandwichform enthält.

7. Ganzkörper-Stereotaxievorrichtung nach Anspruch 1 oder 2, dadurch gekennzeichnet, daß an einem Bügel (10, 12) oder an der Mittelleiste (8) eine Kalibriereinrichtung (20) angebracht ist, die einen definierten Bezugspunkt in der Computertomographie liefert.

8. Ganzkörper-Stereotaxievorrichtung nach Anspruch 7, dadurch gekennzeichnet, daß die Kalibriereinrichtung eine im Querschnitt im wesentlichen dreieckige Lokalisationsplatte (20) aufweist, in der Metalldrähte aufgenommen sind.

9. Ganzkörper-Stereotaxievorrichtung nach einem der vorhergehenden Ansprüche, dadurch gekennzeichnet, daß auf der Grundplatte (2) eine Fixiereinrichtung für den Patienten, insbesondere eine Vakuummatratze aufgenommen ist.

10. Ganzkörper-Stereotaxievorrichtung nach einem der vorhergehenden Ansprüche, dadurch gekennzeichnet, daß Haltebleche (32) vorgesehen sind, die zwischen einer der Randleisten (4, 6) und der Fixierdecke angeordnet werden.

11. Ganzkörper-Stereotaxievorrichtung nach Anspruch 2 dadurch gekennzeichnet, daß eine Lokalisationsplatte (20) an einem Bügel (10, 12) und eine weitere Lokalisationsplatte an der Mittelleiste (8) angebracht sind, wobei die Lokalisationsplatten in senkrechter Richtung miteinander fluchten.

12. Ganzkörper-Stereotaxievorrichtung nach Anspruch 1, dadurch gekennzeichnet, daß an dem Haltestab (26) ein chirurgisches Instrument zur Fixierung der Wirbelsäule des Patienten befestigt ist.

13. Ganzkörper-Stereotaxievorrichtung nach einem der vorhergehenden Ansprüche, dadurch gekennzeichnet, daß ein Bügel (10, 12) zweigeteilt ist.

14. Ganzkörper-Stereotaxievorrichtung nach einem der vorhergehenden Ansprüche, dadurch gekennzeichnet, daß drei zusammenwirkende Zieleinstellungseinrichtungen vorgesehen sind, die an einem Bügel (10, 12) befestigt sind.

15. Ganzkörper-Stereotaxievorrichtung nach einem der vorhergehenden Ansprüche, dadurch gekennzeichnet, daß an einem Bügel (10, 12) ein Hilfsbügel (36) befestigt ist.

16. Ganzkörper-Stereotaxievorrichtung nach Anspruch 15, dadurch gekennzeichnet, daß auf dem Hilfsbügel (36) ein Läufer (44) verschieblich gelagert ist.

## Claims

1. A whole body stereotaxy device with a base plate (2) to hold a patient, two edge strips (4, 6) attached to the edge of the base plate (2) that provide sides to base plate (2) and to which stirrups (10, 12) extending over the base plate (2) can be attached, whereby two neighboring stirrups (10, 12) are attached to the edge strips (4, 6), characterized in that the stirrups (10, 12) are connected by at least one retention rod (26) that is for holding surgical instruments.

2. The whole body stereotaxy device according to claim 1, characterized in that a middle strip (8) is attached to the base plate (2) with holes (22) to receive accessories.

3. The whole body stereotaxy device according to one of claims 1 or 2, characterized in that a stirrup (10, 12) bears a target setting device for later irradiation.

4. The whole body stereotaxy device according to one of claims 1 - 3, characterized in that the base plate contains carbon fibers.

5. The whole body stereotaxy device according to one of the prior claims, characterized in that the base plate contains acrylic foam.

6. The whole body stereotaxy device according to claim 4 and 5, characterized in that the base plate has an alternating acrylic foam layer and a layer of plastic fibers in sandwich form.

7. The whole body stereotaxy device according to claim 1 or 2, characterized in that a calibration device (20) is attached to a stirrup (10, 12) or the middle strip (8) that provides a specific reference point for computerized tomography.

8. The whole body stereotaxy device according to claim 7, characterized in that the calibration device has a localization plate (20) with an essentially triangular cross-section that holds metal wires.

9. The whole body stereotaxy device according to one of the prior claims, characterized in that there is a holding device on the base plate (2) for patients, especially a vacuum mattress.

10. The whole body stereotaxy device according to one of the prior claims, characterized in that retention clips (32) are provided between one of the edge strips (4, 6) and the fixing cover.

11. The whole body stereotaxy device according to claim 2, characterized in that a localization plate (20) is attached to a stirrup (10, 12), and another localization plate is attached to the middle strip (8), whereby the localization plates are flush in a perpendicular direction.

12. The whole body stereotaxy device according to claim 1, characterized in that a surgical instrument is attached to the holding rod (26) to hold the patient's spinal column.

13. The whole body stereotaxy device according to one of the prior claims, characterized in that a stirrup (10, 12) has two parts.

14. The whole body stereotaxy device according to one of the prior claims, characterized in that three interactive target setting devices are provided that are attached to a stirrup (10, 12).

15. The whole body stereotaxy device according to one of the prior claims, characterized in that an auxiliary stirrup (36) is attached to a stirrup (10, 12).

16. The whole body stereotaxy device according to claim 15, characterized in that there is a moveable runner (44) mounted to the auxiliary stirrup (36).

## Revendications

1. Dispositif de stéréotaxie pour le corps entier, comprenant
une plaque d'assise (2) pour réceptionner un patient,
deux rebords (4, 6) fixés sur la plaque d'assise (2) du côté des bords, lesquels assurent la finition latérale de la plaque d'assise (2) et auxquels peuvent être fixés les arceaux (10, 12) enjambant la plaque d'assise (2), deux arceaux (10, 12) en opposition étant disposés sur les rebords (4, 6), caractérisé en ce que les arceaux (10, 12) sont raccordés par au moins une barre de maintien (28) destinée à la fixation d'instruments chirurgicaux.

2. Dispositif de stéréotaxie pour le corps entier selon la revendication 1, caractérisé en ce qu'une latte centrale (8) présentant des percements (22) pour recevoir des accessoires est fixée à la plaque d'assise (2).

3. Dispositif de stéréotaxie pour le corps entier selon l'une des revendications 1 ou 2, caractérisé en ce qu'un des arceaux (10, 12) porte un dispositif de réglage de cible en vue d'une irradiation ultérieure du patient.

4. Dispositif de stéréotaxie pour le corps entier selon l'une des revendications 1 à 3, caractérisé en ce que la plaque d'assise contient des fibres de carbone.

5. Dispositif de stéréotaxie pour le corps entier selon l'une des revendications qui précèdent, caractérisé en ce que la plaque d'assise contient de la mousse acrylique.

6. Dispositif de stéréotaxie pour le corps entier selon les revendications 4 et 5, caractérisé en ce que la plaque d'assise contient en alternance une couche de mousse acrylique et une couche de fibres de carbone en sandwich.

7. Dispositif de stéréotaxie pour le corps entier selon la revendication 1 ou 2, caractérisé en ce qu'un dispositif de calibrage (20) fournissant un point de référence défini en tomographie informatique est placé sur un arceau (10, 12) ou sur la latte centrale (8).

8. Dispositif de stéréotaxie pour le corps entier selon la revendication 7, caractérisé en ce que le dispositif de calibrage présente une plaquette de localisation (20) de section transversale sensiblement triangulaire dans laquelle sont reçus des fils métalliques.

9. Dispositif de stéréotaxie pour le corps entier selon l'une des revendications qui précèdent, caractérisé en ce qu'un dispositif d'immobilisation du patient, en particulier un matelas à vide, est placé sur la plaque d'assise (2).

10. Dispositif de stéréotaxie pour le corps entier selon l'une des revendications qui précèdent, caractérisé en ce qu'on a prévu des tôles de maintien (32) qui sont disposées entre un des rebords (4, 6) et la housse d'immobilisation.

11. Dispositif de stéréotaxie pour le corps entier selon la revendication 2, caractérisé en ce qu'une plaquette de localisation (20) est disposée sur un arceau (10, 12) et une autre plaquette de localisation sur la latte centrale (8), les plaquettes de localisation étant alignées dans le sens vertical.

12. Dispositif de stéréotaxie pour le corps entier selon la revendication 1, caractérisé en ce qu'un instrument chirurgical destiné à l'immobilisation de la colonne vertébrale du patient est fixé sur la barre de maintien (28).

13. Dispositif de stéréotaxie pour le corps entier selon l'une des revendications qui précèdent, caractérisé en c qu'un arceau (10, 12) est divisé en deux.

14. Dispositif de stéréotaxie pour le corps entier selon l'une des revendications qui précèdent, caractérisé en ce qu'on a prévu trois dispositifs de réglage de cible fixés sur un arceau (10, 12).

15. Dispositif de stéréotaxie pour le corps entier selon l'une des revendications qui précèdent, caractérisé en ce qu'un arceau auxiliaire (38) est fixé sur un arceau (10, 12).

16. Dispositif de stéréotaxie pour le corps entier selon la revendication 15, caractérisé en ce qu'un curseur (44) est monté de manière à pouvoir se déplacer sur l'arceau auxiliaire (38).
